# EUROPEAN PATENT SPECIFICATION

(11) **EP 3 344 218 B1**
(45) Date of publication and mention of the grant of the patent: **08.04.2020**
(21) Application number: 17735317.4
(22) Date of filing: 22.06.2017
(51) Int. Cl.: A61K 8/27, A61Q 11/00, A61K 8/19

(54) **ORAL CARE COMPOSITIONS**
MUNDPFLEGEMITTEL
COMPOSITIONS D'HYGIENE BUCCAL

(30) Priority: 24.06.2016 US 201662354258 P
(43) Date of publication of application: 11.07.2018
(73) Proprietor: Colgate-Palmolive Company, New York, NY 10022 (US)
(72) Inventor: THOMSON, Paul, Piscataway, New Jersey 08854 (US); DOGU, Nihal, Dayton, New Jersey 08810 (US); RAJAH, Divino, Long Valley, New Jersey 07853 (US); DAEP, Carlo, Piscataway, NJ 08854 (US); BEGUM-GAFUR, Rehana, Clifton, New Jersey 07011 (US); PRENCIPE, Michael, West Windsor, New Jersey 08550 (US)
(74) Representative: Sonnenhauser, Thomas Martin
(86) International application number: PCT/US2017/038767
(87) International publication number: WO 2017/223321

(56) References cited:
- EP-A1- 2 057 978
- WO-A1-2011/053291
- WO-A1-2015/195139
- WO-A2-2012/087288
- DATABASE GNPD [Online] MINTEL; 1 September 2006 (2006-09-01), "Toothpaste", XP002772848, Database accession no. 586158

## Description

This invention relates to oral care compositions comprising zinc oxide and zinc citrate, a stannous source, and a polyphosphate, as well as to said oral care compositions for use in a method to improve oral health in a subject in need thereof.

### BACKGROUND

Zinc is a known antimicrobial agent used in toothpaste compositions. Zinc is a known essential mineral for human health, and has been reported to help strengthen dental enamel and to promote cell repair.

Stannous ions, in particular stannous salts such as stannous fluoride, are also known anti-microbial agents and are used in various dentifrices as agents for preventing plaque. However, there are certain disadvantages to using stannous salts, such as instability, tendency to stain teeth, astringency, and unpleasant taste for users.

Accordingly, in view of the drawbacks and disadvantages to using various antimicrobials, such as zinc and stannous, there is a need for oral care compositions with anti-bacterial efficacy, but which are also palatable and desirable for a user.

### BRIEF SUMMARY

It has been surprisingly found that the current formulations offer the advantage of robust microbial protection without significantly interfering with the stability of the oral care composition and by allowing for formulations which use comparable amounts of zinc and stannous - which may have undesirable aesthetic qualities (e.g., poor taste) - than certain previous formulations on the market. It is an unexpected benefit that that current formulation use comparable amounts of stannous and zinc than previous formulations, but still maintain or improve the availability of stannous and zinc in the oral cavity of a user. The increased amount of available zinc and stannous aids in protecting against erosion, reducing bacterial colonization and biofilm development.

In one aspect the invention is an oral care composition (Composition 1.0) comprising:
a. a first source of zinc comprising zinc oxide;
b. a second source of zinc comprising zinc citrate;
c. stannous ion source; and
d. a polyphosphate; wherein
   the zinc citrate is in an amount of from 0.25 to 1 wt.% and zinc oxide is present in an amount of from 0.75 to 1.25 wt.% based on the total weight of the composition.

For example, the invention contemplates any of the following compositions (unless otherwise indicated, values are given as percentage of the overall weight of the composition)
1.1 Composition 1.0, wherein the first zinc source is zinc oxide and the second zinc source is zinc citrate.
1.2 Any of the preceding compositions, wherein the wt.% ratio of the amount of zinc oxide to zinc citrate is from 1.5:1 to 4.5:1 (e.g., 2:1, 2.5:1, 3:1, 3.5:1, or 4:1).
1.3 Any of the preceding compositions wherein the zinc citrate is in an amount of 0.5 wt. % and zinc oxide may be present in an amount of 1.0 wt. % based on the weight of the oral care composition.
1.4 Any of the preceding compositions wherein the zinc citrate is about 0.5 wt% (e.g., zinc citrate trihydrate).
1.5 Any of the preceding compositions wherein the zinc oxide is about 1.0 wt%.
1.6 Any of the preceding compositions where the zinc citrate is about 0.5 wt% and the zinc oxide is about 1.0 wt%.
1.7 Composition of 1.0, wherein the zinc oxide is about 1.0 wt%.
1.8 Composition of 1.0, or 1.7, where the zinc citrate is about 0.8 wt% (e.g., about 0.85 wt.%) and the zinc oxide is about 1.0 wt%.
1.9 Any of the preceding compositions, wherein the stannous ion source is stannous fluoride, other stannous halides such as stannous chloride dihydrate, stannous pyrophosphate, organic stannous carboxylate salts such as stannous formate, acetate, gluconate, lactate, tartrate, oxalate, malonate and citrate, stannous ethylene glyoxide, or a mixture thereof.
1.10 Any of the preceding compositions, wherein the stannous source is stannous fluoride.
1.11 Any of the preceding compositions, wherein the stannous fluoride is present in an amount of 0.1 wt. % to 2 wt. % (0.1 wt% - 0.6 wt.%) (e.g., about 0.454 wt.%) of the total composition weight.
1.12 Any of the preceding compositions wherein the stannous fluoride is in an amount from 50 to 25,000 ppm (e.g., 750 -7000ppm, e.g., 1000-5000ppm, e.g., about 4500 ppm, e.g., about 4540ppm).
1.13 Any of the preceding compositions, wherein the composition comprises a copolymer.
1.14 The composition of 1.13, wherein the copolymer is a PVM/MA copolymer.
1.15 The composition of 1.14, wherein the PVM/MA copolymer comprises a 1:4 to 4:1 copolymer of maleic anhydride or acid with a further polymerizable ethylenically unsaturated monomer; for example 1:4 to 4:1, e.g. about 1:1.
1.16 Any of the preceding compositions, wherein the further polymerizable ethylenically unsaturated monomer comprises methyl vinyl ether (methoxyethylene).
1.17 Any of the preceding compositions, wherein the PVM/MA copolymer comprises a copolymer of methyl vinyl ether/maleic anhydride, wherein the anhydride is hydrolyzed following copolymerization to provide the corresponding acid.
1.18 Any of the preceding compositions, wherein the PVM/MA copolymer comprises a GANTREZ® polymer (e.g., GANTREZ® S-97 polymer)
1.19 Any of the preceding compositions wherein the pH is between 7.5 and 10.5. e.g., about 7.5 or about 8.0.
1.20 Any of the preceding compositions further comprising a fluoride ion source.
1.21 The composition of 1.20, wherein the fluoride ion source is selected from the group consisting of stannous fluoride, sodium fluoride, potassium fluoride, sodium monofluorophosphate, sodium fluorosilicate, ammonium fluorosilicate, amine fluoride, ammonium fluoride, and combinations thereof.
1.22 Any of the preceding compositions wherein the polyphosphate is sodium tripolyphosphate (STPP).
1.23 The composition of 1.22, wherein the sodium tripolyphosphate is from 0.5 - 5.0 wt% (e.g., about 3.0 wt%).
1.24 Any of the preceding compositions further comprising an effective amount of one or more alkali phosphate salts, e.g., sodium, potassium or calcium salts, e.g., selected from alkali dibasic phosphate and alkali pyrophosphate salts, e.g., alkali phosphate salts selected from sodium phosphate dibasic, potassium phosphate dibasic, dicalcium phosphate dihydrate, calcium pyrophosphate, tetrasodium pyrophosphate, tetrapotassium pyrophosphate, disodium hydrogenorthophoshpate, monosodium phosphate, pentapotassium triphosphate and mixtures of any of two or more of these, e.g., in an amount of 1-20%, e.g., 2-8%, e.g., ca. 5%>, by weight of the composition.
1.25 Any of the preceding compositions further comprising an abrasive or particulate (e.g., silica).
1.26 The composition of 1.25, wherein the abrasive or particulate is selected from sodium bicarbonate, calcium phosphate (e.g., dicalcium phosphate dihydrate), calcium sulfate, precipitated calcium carbonate, calcium pyrophosphate, silica (e.g., hydrated silica), iron oxide, aluminum oxide, perlite, plastic particles, e.g., polyethylene, and combinations thereof.
1.27 Any of the preceding compositions wherein the silica is synthetic amorphous silica, (e.g., 1% - 25% by wt.) (e.g., 8% - 25% by wt.) (e.g., about 12% by wt.)
1.28 Any of the preceding composition wherein the silica abrasives are silica gels or precipitated amorphous silicas, e.g. silicas having an average particle size ranging from 2.5 microns to 12 microns.
1.29 Any of the preceding compositions further comprising a small particle silica having a median particle size (d50) of 1- 5 microns (e.g., 3 - 4 microns) (e.g., about 5 wt. % Sorbosil AC43 from Ineos Silicas, Warrington, United Kingdom).
1.30 Any of the preceding compositions wherein 20-30 wt% of the total silica in the composition is small particle silica (e.g., having a median particle size (d50) of 3-4 microns) and wherein the small particle silica is about 5 wt.% of the oral care composition.
1.31 Any of the preceding compositions comprising silica wherein the silica is used as a thickening agent, e.g., particle silica.
1.32 Any of the preceding compositions further comprising an anionic surfactant, wherein the nonionic surfactant is in an amount of from 0.5 -5% by wt., e.g, 1-2% by weight, selected from water-soluble salts of higher fatty acid monoglyceride monosulfates, (e.g., sodium N- methyl N-cocoyl taurate), sodium cocomo-glyceride sulfate; higher alkyl sulfates, (e.g., sodium lauryl sulfate); higher alkyl-ether sulfates (e.g., of formula

   CH₃(CH₂)ₘCH₂(OCH₂CH₂)ₙOS0₃X,

   wherein m is 6-16, e.g., 10, n is 1-6, e.g., 2, 3 or 4, and X is Na) or (e.g., sodium laureth-2 sulfate (CH₃(CH2)₁₀CH₂(OCH₂CH₂)₂OS0₃Na); higher alkyl aryl sulfonates (e.g., sodium dodecyl benzene sulfonate, sodium lauryl benzene sulfonate); higher alkyl sulfoacetates (e.g., sodium lauryl sulfoacetate; dodecyl sodium sulfoacetate), higher fatty acid esters of 1,2 dihydroxy propane sulfonate, sulfocolaurate (e.g., N-2- ethyl laurate potassium sulfoacetamide) and sodium lauryl sarcosinate, and mixtures thereof.
1.33 Any of the preceding compositions, wherein the anionic surfactant is sodium lauryl sulfate (e.g., about 1.75 by wt.).
1.34 Any of the preceding compositions further comprising glycerin, wherein the glycerin is in a total amount of 20 - 50% (e.g., about 41% by wt.).
1.35 Any of the preceding compositions, wherein the ratio of the amount of zinc oxide (e.g., wt.%) to zinc citrate (e.g., wt%) is from 1.5:1 to 4.5:1 (e.g., 2:1, 2.5:1, 3:1, 3.5:1, or 4:1).
1.36 Any of the preceding compositions, wherein the zinc citrate is in an amount of 0.5 wt. % and zinc oxide may be present in an amount of 1.0 wt. % based on the weight of the oral care composition.
1.37 Any of the preceding compositions wherein the zinc citrate is about 0.5 wt%.
1.38 Any of the preceding compositions wherein the zinc oxide is about 1.0 wt%.
1.39 Any of the preceding compositions where the zinc citrate is about 0.5 wt% and the zinc oxide is about 1.0 wt%.
1.40 Any of the preceding compositions comprising polymer films.
1.41 Any of the preceding compositions comprising flavoring, fragrance and/or coloring.
1.42 The composition of 1.46, wherein the flavoring agent is sodium saccharin, sucralose, or a mixture thereof.
1.43 Any of the preceding compositions, wherein the composition comprises a thickening agents selected from the group consisting of carboxyvinyl polymers, carrageenan, xanthan, hydroxyethyl cellulose and water soluble salts of cellulose ethers (e.g., sodium carboxymethyl cellulose and sodium carboxymethyl hydroxyethyl cellulose).
1.44 Any of the preceding compositions, wherein the compositions comprises sodium carboxymethyl cellulose (e.g., from 0.1 wt.% - 2.5 wt.%) (e.g., about 0.2% by wt.).
1.45 Any of the preceding compositions comprising from 5% - 40%, e.g., 10% - 35%, e.g., about 10, about 12%, about 15%, about 25%, about 30%, and about 35% water.
1.46 Any of the preceding compositions comprising an additional antibacterial agent selected from halogenated diphenyl ether (e.g. triclosan), herbal extracts and essential oils (e.g., rosemary extract, tea extract, magnolia extract, thymol, menthol, eucalyptol, geraniol, carvacrol, citral, hinokitol, catechol, methyl salicylate, epigallocatechin gallate, epigallocatechin, gallic acid, miswak extract, sea-buckthorn extract), bisguanide antiseptics (e.g., chlorhexidine, alexidine or octenidine), quaternary ammonium compounds (e.g., cetylpyridinium chloride (CPC), benzalkonium chloride, tetradecylpyridinium chloride (TPC), N-tetradecyl-4-ethylpyridinium chloride (TDEPC)), phenolic antiseptics, hexetidine, octenidine, sanguinarine, povidone iodine, delmopinol, salifluor, metal ions (e.g., zinc salts, for example, Zinc Chloride, Zinc Lactate, Zinc Sulfate, stannous salts, copper salts, iron salts), sanguinarine, propolis and oxygenating agents (e.g., hydrogen peroxide, buffered sodium peroxyborate or peroxycarbonate), phthalic acid and its salts, monoperthalic acid and its salts and esters, ascorbyl stearate, oleoyl sarcosine, alkyl sulfate, dioctyl sulfosuccinate, salicylanilide, domiphen bromide, delmopinol, octapinol and other piperidino derivatives, nicin preparations, chlorite salts; and mixtures of any of the foregoing.
1.47 Any of the preceding compositions comprising an antioxidant, e.g., selected from the group consisting of Co-enzyme Q10, PQQ, Vitamin C, Vitamin E, Vitamin A, BHT, anethole-dithiothione, and mixtures thereof.
1.48 Any of the preceding compositions comprising a whitening agent.
1.49 The composition of 1.48, wherein the whitening agent is titanium dioxide.
1.50 Any of the preceding compositions comprising a whitening agent selected from a whitening active selected from the group consisting of peroxides, metal chlorites, perborates, percarbonates, peroxyacids, hypochlorites, and combinations thereof.
1.51 Any of the preceding compositions further comprising hydrogen peroxide or a hydrogen peroxide source, e.g., urea peroxide or a peroxide salt or complex (e.g., such as peroxyphosphate, peroxycarbonate, perborate, peroxysilicate, or persulphate salts; for example calcium peroxyphosphate, sodium perborate, sodium carbonate peroxide, sodium peroxyphosphate, and potassium persulfate), or hydrogen peroxide polymer complexes such as hydrogen peroxide-polyvinyl pyrrolidone polymer complexes.
1.52 Any of the preceding compositions further comprising an agent that interferes with or prevents bacterial attachment, e.g., ELA or chitosan.
1.53 Any of the preceding compositions further a buffer system; (e.g., wherein the buffer comprises trisodium citrate and citric acid).
1.54 Any of the preceding compositions, wherein the composition comprises an aqueous buffer system, for example, wherein the buffer system comprises an organic acid and an alkali metal salt thereof, e.g., wherein the organic acid is citric acid and the salt is a mono-, di- and/or tri- alkali metal citrate salt, e.g., mono-, di- and/or tri- lithium, sodium, potassium, or cesium citrate salt, and citric acid. For example, where the composition comprises 1-10% by weight organic acid salt and 0.1-5% by weight organic acid.
1.55 Composition of 1.54, wherein the buffer system comprises a citrate buffer, wherein the citrate buffer comprises tri-sodium citrate and citric acid (e.g., 1 to 10% by weight of the composition), for example, wherein the molar ratio of mono-, di- and/or tri-sodium citrate and citric acid is 1.5 to 5, (e.g., 2 to 4). The buffer system may be present, by weight, in an amount that is greater than the amount, by weight, of the source of stannous ions.
1.56 Any of the preceding compositions comprising:
   a. about 1.0% zinc oxide
   b. about 0.5% zinc citrate (e.g., zinc citrate trihydrate)
   c. about 0.45% stannous fluoride; and
   d. about 3.0% of sodium tripolyphosphate.
1.57 Any of Composition 1.0 - 1.60, wherein the composition comprises:
   a. about 1.0% zinc oxide
   b. about 0.8% zinc citrate (e.g., zinc citrate trihydrate)
   c. about 0.45% stannous fluoride; and
   d. about 3.0% of sodium tripolyphosphate.
1.58 Any of Composition 1.0 - 1.60, wherein the composition comprises:
   a. about 1.0% zinc oxide
   b. about 0.5% zinc citrate (e.g., zinc citrate trihydrate)
   c. about 4500ppm stannous fluoride; and
   d. about 3.0% of sodium tripolyphosphate.
1.59 Any of Composition 1.0 - 1.60, wherein the composition comprises:
   e. about 1.0% zinc oxide
   f. about 0.5% zinc citrate (e.g., zinc citrate trihydrate)
   g. about 4500ppm stannous fluoride;
   h. about 3.0% of sodium tripolyphosphate; and
   i. about 41% glycerin.
1.60 Any of the preceding compositions further comprising microcrystalline cellulose/sodium carboxymethylcellulose, e.g., in an amount of from 0.1-5%, e.g., 0.5-2%, e.g. 1 %.
1.61 Any of the preceding compositions further comprising polyvinylpyrrolidone (PVP) in an amount of from 0.5-3 wt. %, e.g. about 1.25 wt. %.
1.62 Any of the preceding compositions effective upon application to the oral cavity, e.g., by rinsing, optionally in conjunction with brushing, to (i) reduce or inhibit formation of dental caries, (ii) reduce, repair or inhibit pre-carious lesions of the enamel, e.g., as detected by quantitative light-induced fluorescence (QLF) or electrical caries measurement (ECM), (iii) reduce or inhibit demineralization and promote remineralization of the teeth, (iv) reduce hypersensitivity of the teeth, (v) reduce or inhibit malodor, (vi) promote healing of sores or cuts in the mouth, (vii) reduce levels of acid producing bacteria, (ix) inhibit microbial biofilm formation in the oral cavity, (x) raise and/or maintain plaque pH at levels of at least pH 5.5 following sugar challenge, (xi) reduce plaque accumulation, (xii) treat, relieve or reduce dry mouth, (xiii) clean the teeth and oral cavity (xiv) reduce erosion, (xv) prevents stains and/or whiten teeth, (xvi) immunize the teeth against cariogenic bacteria; and/or (xvii) promote systemic health, including cardiovascular health, e.g., by reducing potential for systemic infection via the oral tissues.
1.63 Any of the preceding oral compositions, wherein the oral composition may be any of the following oral compositions selected from the group consisting of: a toothpaste or a dentifrice, a mouthwash or a mouth rinse, a topical oral gel, a denture cleanser, a coated or impregnated immediate or delayed release oral adhesive strip or patch, and a coated or impregnated oral wipe or swab.
1.64 Any of the preceding compositions, where the only source of zinc is zinc oxide and zinc citrate.
1.65 Any of the preceding compositions, where the only source of stannous is stannous fluoride.
1.66 A composition obtained or obtainable by combining the ingredients as set forth in any of the preceding compositions.
1.67 Any the preceding oral compositions, wherein the composition may be any of the following selected from: a toothpaste, transparent paste, gel, mouth rinse, spray and chewing gum.
1.68 Any of the preceding oral compositions, wherein the composition is incorporated into a chewing gum.

In another embodiment, the invention encompasses the oral care composition of the invention for use in a method to improve oral health comprising applying an effective amount of the oral composition of any of the embodiments (e.g., any of Compositions 1.0 *et seq*) set forth above to the oral cavity of a subject in need thereof, in particular to
i. reduce or inhibit formation of dental caries,
ii. reduce levels of acid producing bacteria,
iii. inhibit microbial bio film formation in the oral cavity,
iv. reduce plaque accumulation,
v. immunize (or protect) the teeth against cariogenic bacteria and their effects, and/or
vi. clean the teeth and oral cavity.

Sodium bicarbonate, sodium methyl cocoyl taurate (tauranol), MIT, and benzyl alcohol and combinations thereof can be used in the manufacture of a composition of the invention.

### DETAILED DESCRIPTION

As used herein, the term "oral composition" means the total composition that is delivered to the oral surfaces. The composition is further defined as a product which, during the normal course of usage, is not, the purposes of systemic administration of particular therapeutic agents, intentionally swallowed but is rather retained in the oral cavity for a time sufficient to contact substantially all of the dental surfaces and/or oral tissues for the purposes of oral activity. Examples of such compositions include, but are not limited to, toothpaste or a dentifrice, a mouthwash or a mouth rinse, a topical oral gel, a denture cleanser, and the like.

As used herein, the term "dentifrice" means paste, gel, or liquid formulations unless otherwise specified. The dentifrice composition can be in any desired form such as deep striped, surface striped, multi-layered, having the gel surrounding the paste, or any combination thereof. Alternatively the oral composition may be dual phase dispensed from a separated compartment dispenser.

### Fluoride Ion Source

The oral care compositions may further include one or more fluoride ion sources, e.g., soluble fluoride salts. A wide variety of fluoride ion-yielding materials can be employed as sources of soluble fluoride in the present compositions. Examples of suitable fluoride ion-yielding materials are found in U.S. Pat. No. 3,535,421, to Briner et al.; U.S. Pat. No. 4,885,155, to Parran, Jr. et al. and U.S. Pat. No. 3,678,154, to Widder et al.. Representative fluoride ion sources used with the present invention (e.g., Composition 1.0 *et seq*.) include, but are not limited to, stannous fluoride, sodium fluoride, potassium fluoride, sodium monofluorophosphate, sodium fluorosilicate, ammonium fluorosilicate, amine fluoride, ammonium fluoride, and combinations thereof. In certain embodiments the fluoride ion source includes stannous fluoride, sodium fluoride, sodium monofluorophosphate as well as mixtures thereof. Where the formulation comprises calcium salts, the fluoride salts are preferably salts wherein the fluoride is covalently bound to another atom, e.g., as in sodium monofluorophosphate, rather than merely ionically bound, e.g., as in sodium fluoride.

### Surfactants

The invention may in some embodiments contain anionic surfactants, e.g., the Compositions of Composition 1.0, *et seq*., for example, water-soluble salts of higher fatty acid monoglyceride monosulfates, such as the sodium salt of the monosulfated monoglyceride of hydrogenated coconut oil fatty acids such as sodium N- methyl N-cocoyl taurate, sodium cocomo-glyceride sulfate; higher alkyl sulfates, such as sodium lauryl sulfate; higher alkyl-ether sulfates, e.g., of formula

CH₃(CH₂)ₘCH₂(OCH₂CH₂)ₙOS0₃X,

wherein m is 6-16, e.g., 10, n is 1-6, e.g., 2, 3 or 4, and X is Na or , for example sodium laureth-2 sulfate (CH₃(CH2)₁₀CH₂(OCH₂CH₂)₂OS0₃Na); higher alkyl aryl sulfonates such as sodium dodecyl benzene sulfonate (sodium lauryl benzene sulfonate); higher alkyl sulfoacetates, such as sodium lauryl sulfoacetate (dodecyl sodium sulfoacetate), higher fatty acid esters of 1,2 dihydroxy propane sulfonate, sulfocolaurate (N-2- ethyl laurate potassium sulfoacetamide) and sodium lauryl sarcosinate. By "higher alkyl" is meant, e.g., C₆-₃o alkyl. In particular embodiments, the anionic surfactant (where present) is selected from sodium lauryl sulfate and sodium ether lauryl sulfate. When present, the anionic surfactant is present in an amount which is effective, e.g., > 0.001% by weight of the formulation, but not at a concentration which would be irritating to the oral tissue, e.g., 1 %, and optimal concentrations depend on the particular formulation and the particular surfactant. In one embodiment, the anionic surfactant is present at from 0.03% to 5% by weight, e.g., about 1.75% by wt.

In another embodiment, cationic surfactants useful in the present invention can be broadly defined as derivatives of aliphatic quaternary ammonium compounds having one long alkyl chain containing 8 to 18 carbon atoms such as lauryl trimethylammonium chloride, cetyl pyridinium chloride, cetyl trimethylammonium bromide, di-isobutylphenoxyethyldimethylbenzylammonium chloride, coconut alkyltrimethylammonium nitrite, cetyl pyridinium fluoride, and mixtures thereof. Illustrative cationic surfactants are the quaternary ammonium fluorides described in U.S. Pat. No. 3,535,421, to Briner et al.. Certain cationic surfactants can also act as germicides in the compositions.

Illustrative nonionic surfactants of Composition 1.0, *et seq*., that can be used in the compositions of the invention can be broadly defined as compounds produced by the condensation of alkylene oxide groups (hydrophilic in nature) with an organic hydrophobic compound which may be aliphatic or alkylaromatic in nature. Examples of suitable nonionic surfactants include, but are not limited to, the Pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides and mixtures of such materials. In a particular embodiment, the composition of the invention comprises a nonionic surfactant selected from polaxamers (e.g., polaxamer 407), polysorbates (e.g., polysorbate 20), polyoxyl hydrogenated castor oils (e.g., polyoxyl 40 hydrogenated castor oil), and mixtures thereof.

Illustrative amphoteric surfactants of Composition 1.0, *et seq.,* that can be used in the compositions of the invention include betaines (such as cocamidopropylbetaine), derivatives of aliphatic secondary and tertiary amines in which the aliphatic radical can be a straight or branched chain and wherein one of the aliphatic substituents contains about 8-18 carbon atoms and one contains an anionic water-solubilizing group (such as carboxylate, sulfonate, sulfate, phosphate or phosphonate), and mixtures of such materials.

The surfactant or mixtures of compatible surfactants can be present in the compositions of the present invention in 0.1% to 5%, in another embodiment 0.3% to 3% and in another embodiment 0.5% to 2% by weight of the total composition.

### Flavoring Agents

The oral care compositions of the invention may also include a flavoring agent. Flavoring agents which are used in the practice of the present invention include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. Certain embodiments employ the oils of peppermint and spearmint.

The flavoring agent is incorporated in the oral composition at a concentration of 0.01 to 1% by weight.

### pH Adjusting Agents

In some embodiments, the compositions of the present disclosure contain a buffering agent. Examples of buffering agents include anhydrous carbonates such as sodium carbonate, sesquicarbonates, bicarbonates such as sodium bicarbonate, silicates, bisulfates, phosphates (e.g., monopotassium phosphate, monosodium phosphate, disodium phosphate, dipotassium phosphate, tribasic sodium phosphate, sodium tripolyphosphate, pentapotassium tripolyphosphate, phosphoric acid), citrates (e.g. citric acid, trisodium citrate dehydrate), pyrophosphates (sodium and potassium salts, e.g., tetrapotassium pyrophosphate) and combinations thereof. The amount of buffering agent is sufficient to provide a pH of about 5 to about 9, preferable about 6 to about 8, and more preferable about 7, when the composition is dissolved in water, a mouthrinse base, or a toothpaste base. Typical amounts of buffering agent are about 5% to about 35%, in one embodiment about 10% to about 30%, in another embodiment about 15% to about 25%, by weight of the total composition.

### Chelating and anti-calculus agents

The oral care compositions of the invention also may include one or more chelating agents able to complex calcium found in the cell walls of the bacteria. Binding of this calcium weakens the bacterial cell wall and augments bacterial lysis.

Another group of agents suitable for use as chelating or anti-calculus agents in the present invention are the soluble pyrophosphates. The pyrophosphate salts used in the present compositions can be any of the alkali metal pyrophosphate salts. In certain embodiments, salts include tetra alkali metal pyrophosphate, dialkali metal diacid pyrophosphate, trialkali metal monoacid pyrophosphate and mixtures thereof, wherein the alkali metals are sodium or potassium. The salts are useful in both their hydrated and unhydrated forms. An effective amount of pyrophosphate salt useful in the present composition is generally enough to provide at least 0.1 wt. % pyrophosphate ions, e.g., 0.1 to 3 wt.%, e.g., 0.1 to 2 wt. %, e.g., 0.1 to 1 wt.%, e.g., 0.2 to 0.5 wt%. The pyrophosphates also contribute to preservation of the compositions by lowering water activity.

Suitable anticalculus agents for the invention (e.g., Composition 1.0 *et seq)* include without limitation phosphates and polyphosphates (for example pyrophosphates), polyaminopropanesulfonic acid (AMPS), hexametaphosphate salts, zinc citrate trihydrate, polypeptides, polyolefin sulfonates, polyolefin phosphates, diphosphonates. In particular embodiments, the invention includes alkali phosphate salts, i.e., salts of alkali metal hydroxides or alkaline earth hydroxides, for example, sodium, potassium or calcium salts. "Phosphate" as used herein encompasses orally acceptable mono- and polyphosphates, for example, P₁₋₆ phosphates, for example monomeric phosphates such as monobasic, dibasic or tribasic phosphate; dimeric phosphates such as pyrophosphates; and multimeric phosphates, e.g., sodium hexametaphosphate. In particular examples, the selected phosphate is selected from alkali dibasic phosphate and alkali pyrophosphate salts, e.g., selected from sodium phosphate dibasic, potassium phosphate dibasic, dicalcium phosphate dihydrate, calcium pyrophosphate, tetrasodium pyrophosphate, tetrapotassium pyrophosphate, sodium tripolyphosphate, and mixtures of any of two or more of these. In a particular embodiment, for example the compositions comprise a mixture of tetrasodium pyrophosphate (Na₄P₂O₇), calcium pyrophosphate (Ca₂P₂O₇), and sodium phosphate dibasic (Na₂HPO₄), e.g., in amounts of ca. 3-4% of the sodium phosphate dibasic and ca. 0.2-1 % of each of the pyrophosphates. In another embodiment, the compositions comprise a mixture of tetrasodium pyrophosphate (TSPP) and sodium tripolyphosphate (STPP)(Na₅P₃O₁₀), e.g., in proportions of TSPP at about 1-2% and STPP at about 7% to about 10%. Such phosphates are provided in an amount effective to reduce erosion of the enamel, to aid in cleaning the teeth, and/or to reduce tartar buildup on the teeth, for example in an amount of 2-20%, e.g., ca. 5-15%, by weight of the composition.

### Polymers

The oral care compositions of the invention also optionally include one or more polymers, such as polyethylene glycols, polyvinyl methyl ether maleic acid copolymers, polysaccharides (e.g., cellulose derivatives, for example carboxymethyl cellulose, or polysaccharide gums, for example xanthan gum or carrageenan gum). Acidic polymers, for example polyacrylate gels, may be provided in the form of their free acids or partially or fully neutralized water soluble alkali metal (e.g., potassium and sodium) or ammonium salts. Certain embodiments include 1:4 to 4:1 copolymers of maleic anhydride or acid with another polymerizable ethylenically unsaturated monomer, for example, methyl vinyl ether (methoxyethylene) having a molecular weight (M.W.) of about 30,000 to about 1,000,000. These copolymers are available for example as Gantrez AN 139(M.W. 500,000), AN 1 19 (M.W. 250,000) and S-97 Pharmaceutical Grade (M.W. 70,000), of GAF Chemicals Corporation.

Other operative polymers include those such as the 1:1 copolymers of maleic anhydride with ethyl acrylate, hydroxyethyl methacrylate, N-vinyl-2-pyrollidone, or ethylene, the latter being available for example as Monsanto EMA No. 1 103, M.W. 10,000 and EMA Grade 61, and 1 : 1 copolymers of acrylic acid with methyl or hydroxyethyl methacrylate, methyl or ethyl acrylate, isobutyl vinyl ether or N-vinyl-2-pyrrolidone.

The N-vinyl-2-pyrrolidione is also commonly known as polyvinylpyrrolidone or "PVP". PVP refers to a polymer containing vinylpyrrolidone (also referred to as N-vinylpyrrnlidone and N-vinyl-2-pyrrolidinone) as a monomeric unit. The monomeric unit consists of a polar imide group, four non-polar methylene groups and a non-polar methane group. The polymers include soluble and insoluble homopolymeric PVPs. Copolymers containing PVP include vinylpyrrolidone/vinyl acetate (also known as Copolyvidone, Copolyvidonum or VP-VAc) and vinyl pyrrolidone/dimethylamino-ethylmethacrylate. Soluble PVP polymers among those useful herein are known in the art, including Povidone, Polyvidone, Polyvidonum, poly(N-vinyl-2-pyrrolidinone), poly (N-vinylbutyrolactam), poly(1-vinyl-2-pyrrolidone) and poly [1-(2-oxo-1pyrrolidinyl)ethylene]. These PVP polymers are not substantially cross-linked. In some embodiments the polymer comprises an insoluble cross-linked homopolymer. Such polymers include crosslinked PVP (often referred to as cPVP, polyvinylpolypyrrolidone, or cross-povidone).

Suitable generally, are polymerized olefinically or ethylenically unsaturated carboxylic acids containing an activated carbon-to-carbon olefinic double bond and at least one carboxyl group, that is, an acid containing an olefinic double bond which readily functions in polymerization because of its presence in the monomer molecule either in the alpha-beta position with respect to a carboxyl group or as part of a terminal methylene grouping. Illustrative of such acids are acrylic, methacrylic, ethacrylic, alpha-chloroacrylic, crotonic, beta-acryloxy propionic, sorbic, alpha-chlorsorbic, cinnamic, beta-styrylacrylic, muconic, itaconic, citraconic, mesaconic, glutaconic, aconitic, alpha-phenylacrylic, 2-benzyl acrylic, 2-cyclohexylacrylic, angelic, umbellic, fumaric, maleic acids and anhydrides. Other different olefinic monomers copolymerizable with such carboxylic monomers include vinylacetate, vinyl chloride, dimethyl maleate and the like. Copolymers contain sufficient carboxylic salt groups for water-solubility.

A further class of polymeric agents includes a composition containing homopolymers of substituted acrylamides and/or homopolymers of unsaturated sulfonic acids and salts thereof, in particular where polymers are based on unsaturated sulfonic acids selected from acrylamidoalykane sulfonic acids such as 2-acrylamide 2 methylpropane sulfonic acid having a molecular weight of about 1,000 to about 2,000,000, described in U.S. Pat. No. 4,842,847, Jun. 27, 1989 to Zahid.

In preparing oral care compositions, it is sometimes necessary to add some thickening material to provide a desirable consistency or to stabilize or enhance the performance of the formulation. In certain embodiments, the thickening agents are carboxyvinyl polymers, carrageenan, xanthan, hydroxyethyl cellulose and water soluble salts of cellulose ethers such as sodium carboxymethyl cellulose and sodium carboxymethyl hydroxyethyl cellulose. Natural gums such as karaya, gum arabic, and gum tragacanth can also be incorporated. Colloidal magnesium aluminum silicate or finely divided silica can be used as component of the thickening composition to further improve the composition's texture. In certain embodiments, thickening agents in an amount of about 0.5% to about 5.0% by weight of the total composition are used.

In some embodiments, microcrystalline cellulose (MCC) can be used (e.g., carboxymethyl cellulose with sodium carboxymethyl cellulose). An example of a source of MCC is Avicel ® (FMC Corporation), which contains MCC in combination with sodium carboxymethyl cellulose (NaCMC). Both Avicel ®. RC-591 (MCC containing 8.3 to 13.8 weight % NaCMC) and Avicel ®. CL-611 (MCC containing 11.3 to 18.8 weight % NaCMC) may be used in certain aspects. In certain embodiments, the ratio of microcrystalline cellulose to cellulose ether thickening agent is from 1:1 to 1:3 by weight; or from 1:1.5 to 1:2.75 by weight. In any of the above embodiments comprising sodium carboxymethylcellulose, microcrystalline cellulose may be used in combination with NaCMC. In certain such embodiments, the MCC/sodium carboxymethylcellulose may be present in an amount of from 0.5 to 1.5 weight % based on the total weight of the composition.

### Abrasives

Natural calcium carbonate is found in rocks such as chalk, limestone, marble and travertine. It is also the principle component of egg shells and the shells of mollusks. The natural calcium carbonate abrasive of the invention is typically a finely ground limestone which may optionally be refined or partially refined to remove impurities. For use in the present invention, the material has an average particle size of less than 10 microns, e.g., 3-7 microns, e.g. about 5.5 microns. For example a small particle silica may have an average particle size (D50) of 2.5 - 4.5 microns. Because natural calcium carbonate may contain a high proportion of relatively large particles of not carefully controlled, which may unacceptably increase the abrasivity, preferably no more than 0.01%, preferably no more than 0.004% by weight of particles would not pass through a 325 mesh. The material has strong crystal structure, and is thus much harder and more abrasive than precipitated calcium carbonate. The tap density for the natural calcium carbonate is for example between 1 and 1.5 g/cc, e.g., about 1.2 for example about 1.19 g/cc. There are different polymorphs of natural calcium carbonate, e.g., calcite, aragonite and vaterite, calcite being preferred for purposes of this invention. An example of a commercially available product suitable for use in the present invention includes Vicron ® 25-11 FG from GMZ.

Precipitated calcium carbonate is generally made by calcining limestone, to make calcium oxide (lime), which can then be converted back to calcium carbonate by reaction with carbon dioxide in water. Precipitated calcium carbonate has a different crystal structure from natural calcium carbonate. It is generally more friable and more porous, thus having lower abrasivity and higher water absorption. For use in the present invention, the particles are small, e.g., having an average particle size of 1 - 5 microns, and e.g., no more than 0.1 %, preferably no more than 0.05% by weight of particles which would not pass through a 325 mesh. The particles may for example have a D50 of 3-6 microns, for example 3.8=4.9, e.g., about 4.3; a D50 of 1-4 microns, e.g. 2.2-2.6 microns, e.g., about 2.4 microns, and a D10 of 1-2 microns, e.g., 1.2-1.4, e.g. about 1.3 microns. The particles have relatively high water absorption, e.g., at least 25 g/100g, e.g. 30-70 g/100g. Examples of commercially available products suitable for use in the present invention include, for example, Carbolag® 15 Plus from Lagos Industria Quimica.

In certain embodiments the invention may comprise additional calcium-containing abrasives, for example calcium phosphate abrasive, e.g., tricalcium phosphate (Ca₃(P0₄)₂), hydroxyapatite (Ca₁₀(P0₄)₆(OH)₂), or dicalcium phosphate dihydrate (CaHP0₄ · 2H₂0, also sometimes referred to herein as DiCal) or calcium pyrophosphate, and/or silica abrasives, sodium metaphosphate, potassium metaphosphate, aluminum silicate, calcined alumina, bentonite or other siliceous materials, or combinations thereof. Any silica suitable for oral care compositions may be used, such as precipitated silicas or silica gels. For example synthetic amorphous silica. Silica may also be available as a thickening agent, e.g., particle silica. For example, the silica can also be small particle silica (e.g., Sorbosil AC43 from PQ Corporation, Warrington, United Kingdom). However the additional abrasives are preferably not present in a type or amount so as to increase the RDA of the dentifrice to levels which could damage sensitive teeth, e.g., greater than 130.

### Water

Water is present in the oral compositions of the invention. Water, employed in the preparation of commercial oral compositions should be deionized and free of organic impurities. Water commonly makes up the balance of the compositions and includes 5% to 45%, e.g., 10% to 20%, e.g., 25 - 35%, by weight of the oral compositions. This amount of water includes the free water which is added plus that amount which is introduced with other materials such as with sorbitol or silica or any components of the invention. The Karl Fischer method is a one measure of calculating free water.

### Humectants

Within certain embodiments of the oral compositions, it is also desirable to incorporate a humectant to reduce evaporation and also contribute towards preservation by lowering water activity. Certain humectants can also impart desirable sweetness or flavor to the compositions. The humectant, on a pure humectant basis, generally includes 15% to 70% in one embodiment or 30% to 65% in another embodiment by weight of the composition.

Suitable humectants include edible polyhydric alcohols such as glycerin, sorbitol, xylitol, propylene glycol as well as other polyols and mixtures of these humectants. Mixtures of glycerin and sorbitol may be used in certain embodiments as the humectant component of the compositions herein.

The present invention in its method aspect involves applying to the oral cavity a safe and effective amount of the compositions described herein.

The compositions and methods according to the invention (e.g., Composition 1.0 *et seq*) can be incorporated into oral compositions for the care of the mouth and teeth such as dentifrices, toothpastes, transparent pastes, gels, mouth rinses, sprays and chewing gum.

As used throughout, ranges are used as shorthand for describing each and every value that is within the range. Any value within the range can be selected as the terminus of the range. It is understood that when formulations are described, they may be described in terms of their ingredients, as is common in the art, notwithstanding that these ingredients may react with one another in the actual formulation as it is made, stored and used, and such products are intended to be covered by the formulations described.

The following examples further describe and demonstrate illustrative embodiments within the scope of the present invention. The examples are given solely for illustration and are not to be construed as limitations of this invention

### EXAMPLES

### Example 1

Table 1 demonstrates that Test Formulations of the present invention with 0.454% SnF₂, 1.0% ZnO and 0.5% Zn Citrate, and 3% Sodium Tripolyphosphate (STPP) demonstrate improved stannous and zinc uptake in 1.0" HAP disc assays (e.g., an enamel mimic) when compared to formulations which contain SnF₂ and zinc lactate (but not ZnO and Zn Citrate)). Values below are normalized to the negative control formulations (e.g., Baseline = "1").

**Table 1**

| | Total Stannous uptake (ppm)* | Total Zinc uptake (ppm)* |
|---|---|---|
| Control Formulation (SnF₂ and zinc lactate) | 1.0 | 1.0 |
| Test Formulation (SnF₂, ZnCit, ZnO, STPP) | 2.29 | 1.94 |

| | | |
|---|---|---|
| * Values normalized to untreated negative control (Baseline = "1") | | |

### Example 2 (not claimed)

Table 2 demonstrates that that test Formulations with 0.454% SnF₂, 1.0% ZnO and 0.8% Zn Citrate, demonstrate improved zinc uptake, and maintained stannous uptake, in 1.0" HAP disc assays (e.g., an enamel mimic), when compared to control formulations which contain SnF₂ and zinc lactate (but not ZnO and Zn Citrate). Values below are normalized to the negative control formulations (e.g., Baseline = "1"):

**Table 2**

| | Total Stannous uptake (ppm)* | Total Zinc uptake (ppm)* |
|---|---|---|
| Control Formulation (SnF₂ and zinc lactate) | 100 | 300 |
| Test Formulation (SnF₂, ZnCit, ZnO) | About 175 | About 900 |

| | | |
|---|---|---|
| * Values normalized to untreated negative control (Baseline = "1") | | |

### Example 3

Table 3 demonstrates that test Formulations of the present invention with 0.454% SnF₂, 1.0% ZnO and 0.5% Zn Citrate, and 3% Sodium Tripolyphosphate (STPP) demonstrated improved zinc uptake in Vitroskin assays and maintained stannous uptake, when compared to formulations which contain SnF₂ and zinc lactate (but not ZnO and Zn Citrate). The Vitroskin assay method involves incubating 20mm diameter VitroSkins in saliva followed by treatment with a 1:2 slurry of toothpaste and water for 2 mins, then washing with deionized water. The Vitroskins were then dissolved completely with 1mL of concentrated nitric acid, then diluted with 9mL of deionized water, filtered, and the dissolved solution obtained submitted for analytical analysis of Sn and Zn. Values below are normalized to the negative control formulations (e.g., Baseline = "1").

**Table 3**

| | Total Sn uptake (µg/cm²)* | Total Zinc uptake(µg/cm²)* |
|---|---|---|
| Control Formulation (SnF₂ and zinc lactate) | 1.0 | 1.0 |
| Test Formulation (SnF₂, ZnCit, ZnO, STPP) | .90 | 4.00 |

| | | |
|---|---|---|
| * Values normalized to untreated negative control (Baseline = "1") | | |

### Example 4

Table 4 demonstrates that Test Formulations with 0.454% SnF₂, 1.0% ZnO and 0.5% Zn Citrate, and 3% Sodium Tripolyphosphate (STPP) demonstrated improved zinc uptake, and maintained stannous uptake, in bovine enamel assays when compared to control formulations which contain SnF₂ and zinc lactate (but not ZnO and Zn Citrate). Values below are normalized to the negative control formulations (e.g., Baseline = "1"):

**Table 4**

| | Total Sn uptake (ppm)* | Total Zinc uptake (ppm)* |
|---|---|---|
| Control Formulation (SnF₂ and zinc lactate) | 1.0 | 1.0 |
| Test Formulation (SnF₂, ZnCit, ZnO, and STPP) | 1.01 | 2.19 |

| | | |
|---|---|---|
| * Values normalized to untreated negative control (Baseline = "1") | | |

### Example 5

### Planktonic: ATP Assay

Table 5 demonstrates an ATP assay which shows cells viability after treatment with dentifrices. Overall the Sn dual zinc technology provides increased metal ion uptake, and which seemingly correlates with dentifrice efficacy in biological ATP assays that show an increased reduction in bacteria cells (i.e., lower percentage of viable bacterial cells) when compared to formulations which contain SnF and zinc lactate (but not ZnO, Zn Citrate, or Sodium Tripolyphosphate (STPP)):

**Table 5**

| Sample | % Viability |
|---|---|
| Placebo | 47.54% |
| Control Formulation (SnF₂ and zinc lactate) | 24.98% |
| Test Formulation (SnF₂, ZnCit, ZnO) | 6.54% |

### Example 6

It was further investigated that in a biofilm assay there was a lower pH change (i.e., a lower pH change is considered better and can be indicative of how much acid the biofilm is producing) for formulations comprising SnF₂, ZnCit, ZnO, compared to: a) negative control which is a NaF placebo, and b) formulations comprising SnF₂ & Zinc Lactate (but no additional Zn oxide or Zn citrate) (not claimed).

There is also a higher reduction in ATP production in samples treated with formulations comprising SnF₂, Zn Citrate, ZnO, compared to: a) negative control which is a NaF placebo, and b) formulations comprising SnF₂ & Zinc Lactate (but no additional Zn oxide or Zn citrate) (not claimed). The ability to reduce ATP production can be indicative of lower bio-film metabolic activity.

### Example 7

### Representative Formulas (values are % wt. of composition)

**Table 6**

| **Description** | **Weight %** |
|---|---|
| Humectant | 35% - 50% |
| Abrasive | 16.0% - 20.0% |
| pH Adjusting Agent | 5% - 9% |
| Polymer | 4% - 7% |
| Colorant, Flavoring Agent | 2.0% - 2.5% |
| Amphoteric Surfactant | 0.5% - 2.0% |
| Thickening agent | 1.0% - 2.0% |
| Stannous Fluoride, USP | 0.4% - 0.5% |
| Demineralized Water | Q.S. |
| Nonionic Surfactant | 1.0% - 2.5% |
| Zinc Oxide | 1 |
| Alkali Phosphate Salt | 0.5% - 2.0% |
| 85% Syrupy Phosphoric Acid - Food Grade | 0.25% - 0.75% |
| Zinc Citrate Trihydrate | 0.5 |
| Total Components | 100 |

### Example 8

### Representative Formulations

**Table 7 (not claimed)**

| **Description** | **Weight %** |
|---|---|
| Demineralized Water | Q.S. |
| Colorant, Flavoring Agents | 0.5 - 2.0% |
| pH Adjusting Agents | 5% - 8% |
| Stannous Fluoride, USP | 0.45 |
| Zinc L-lactate dihydrate | 2.5 |
| Zinc Oxide | 0.5 |
| Alkali Phosphate Salt | 1% - 3% |
| Humectants | 30% - 50% |
| Polymer | 3% -6% |
| Thickeners | 1% -3% |
| Abrasives | 20% - 30% |
| Nonionic Surfactant | 1% -2% |
| Amphoteric Surfactant | .5% - 2% |
| Total Components | 100% |

**Table 8**

| **Description** | **Weight (%)** |
|---|---|
| DEMINERALIZED WATER | Q.S. |
| Colorant, Flavoring Agents | 2% -4% |
| pH Adjusting Agents | 3% - 5% |
| Stannous Fluoride, USP | 0.45 |
| Zinc Citrate Trihydrate | 0.8 |
| Zinc Oxide | 1 |
| Alkali Phosphate Salt | 1% - 3% |
| Polyphosphate | 2% - 4% |
| Humectant | 40% - 50% |
| Polymer | 3% -6% |
| Thickener | 1% -3% |
| Abrasives | 20% -30% |
| Nonionic Surfactant | 1% -3% |
| Amphoteric Surfactant | 0.5% -2.0% |
| Total Components | 100% |

### Example 9

### Representative Formula (values are % wt. of composition)

**Table 9**

| **Description** | **Weight %** |
|---|---|
| Humectant | 35% - 50% |
| Abrasive | 20.0% - 30.0% |
| pH Adjusting Agent | 5% - 9% |
| Polymer | 5% - 8% |
| Colorant, Flavoring Agent | 2.0% - 3.5% |
| Amphoteric Surfactant | 0.5% - 2.0% |
| Thickening agent | 0.1% - 2.0% |
| Stannous Fluoride, USP | 0.454% |
| Demineralized Water | Q.S. |
| Nonionic Surfactant | 1.0% - 2.5% |
| Zinc Oxide | 1.0 |
| Alkali Phosphate Salt | 0.5% - 2.0% |
| 85% Syrupy Phosphoric Acid - Food Grade | 0.25% - 0.75% |
| Zinc Citrate Trihydrate | 0.5 |
| Total Components | 100 |

As used throughout, ranges are used as shorthand for describing each and every value that is within the range. Any value within the range can be selected as the terminus of the range. In addition, all references cited herein are hereby incorporated by referenced in their entireties. In the event of a conflict in a definition in the present disclosure and that of a cited reference, the present disclosure controls.

Unless otherwise specified, all percentages and amounts expressed herein and elsewhere in the specification should be understood to refer to percentages by weight. The amounts given are based on the active weight of the material.

## Claims

1. An oral care composition comprising:
a. a first source of zinc comprising zinc oxide;
b. a second source of zinc comprising zinc citrate;
c. stannous ion source; and
d. a polyphosphate; wherein
the zinc citrate is in an amount of from 0.25 to 1 wt.% and zinc oxide is present in an amount of from 0.75 to 1.25 wt.% based on the total weight of the composition.

2. The oral care composition of any of the preceding claims, wherein the stannous ion source is stannous fluoride, stannous chloride dihydrate, stannous pyrophosphate, organic stannous carboxylate salts such as stannous formate, acetate, gluconate, lactate, tartrate, oxalate, malonate and citrate, or a mixture thereof.

3. The oral care composition of claim 1, wherein the stannous source is stannous fluoride, preferably
wherein the stannous fluoride is present in an amount of 0.1 wt. % to 2 wt. % based on the total weight of the composition, or
wherein the stannous fluoride is in an amount from 50 to 25,000 ppm.

4. The oral care composition of any of the preceding claims, wherein composition comprises a copolymer.

5. The oral care composition of claim 4, wherein the copolymer is a PVM/MA copolymer.

6. The oral care composition of any of the preceding claims, wherein the polyphosphate is sodium tripolyphosphate.

7. The oral care composition of claim 6, wherein the sodium tripolyphosphate is present in an amount of from 0.5 - 5.0 wt%, based on the total weight of the composition.

8. The oral care composition of any of the preceding claims, wherein the composition further comprises glycerin, wherein the glycerin is in a total amount of 20 - 50%, based on the total weight of the composition.

9. The oral care composition of any of the preceding claims, wherein the ratio of the amount of zinc oxide (wt.%) to zinc citrate (wt%) is from 1.5:1 to 4.5:1.

10. The oral care composition of any of the preceding claims comprising a whitening agent.

11. The oral care composition of any of the preceding claims comprising:
a. about 1.0% zinc oxide
b. about 0.5% zinc citrate
c. about 4500ppm stannous fluoride;
d. about 3.0% of sodium tripolyphosphate

12. Any of the preceding compositions comprising:
e. about 1.0% zinc oxide
f. about 0.5% zinc citrate
g. about 4500ppm stannous fluoride;
h. about 3.0% of sodium tripolyphosphate; and
i. about 41% glycerin

13. The oral care composition of any of the preceding claims, wherein the oral composition may be any of the following oral compositions selected from the group consisting of: a toothpaste or a dentifrice, a mouthwash or a mouth rinse, a topical oral gel, a chewing gum, and a denture cleanser.

14. The oral care composition of any of the preceding claims, wherein the composition is obtained or obtainable by combining the ingredients as set forth in any of the preceding compositions.

15. Oral care composition of any of the preceding claims for use in a method to improve oral health comprising applying an effective amount of said oral care composition to the oral cavity of a subject in need thereof.

## Patentansprüche

1. Mundpflegezusammensetzung, umfassend:
a. eine erste Zinkquelle, die Zinkoxid umfasst;
b. eine zweite Zinkquelle, die Zinkcitrat umfasst;
c. Zinn(II)-Ionenquelle; und
d. ein Polyphosphat; wobei
e. das Zinkcitrat in einer Menge von 0,25 bis 1 Gew.-% und Zinkoxid in einer Menge von 0,75 bis 1,25 Gew.-% vorliegt, bezogen auf das Gesamtgewicht der Zusammensetzung.

2. Mundpflegezusammensetzung nach einem der vorhergenden Ansprüche, wobei die Zinn(II)-Ionenquelle Zinn(II)-fluorid, Zinn(II)-chlorid-Dihydrat, Zinn(II)-pyrophosphat, organische Zinn(ii)-carboxylat-Salze wie Zinn(II)-format, -acetat, - gluconat, -lactat, -tartrat, -oxalat, -malonat und -citrat oder ein Gemisch aus denselben ist.

3. Mundpflegezusammensetzung nach Anspruch 1, wobei die Zinn(II)-Quelle Zinn(II)-fluorid ist, bevorzugt
wobei das Zinn(II)-fluorid in einer Menge von 0,1 Gew.-% bis 2 Gew.-%
bezogen auf das Gesamtgewicht der Zusammensetzung vorliegt; oder
wobei das Zinn(II)-fluorid in einer Menge von 50 bis 25.000 ppm vorliegt.

4. Mundpflegezusammensetzung nach einem der vorhergehenden Ansprüche, wobei die Zusammensetzung ein Copolymer umfasst.

5. Mundpflegezusammensetzung nach Anspruch 4, wobei das Copolymer ein PVM/MA-Copolymer ist.

6. Mundpflegezusammensetzung nach einem der vorhergehenden Ansprüche, wobei das Polyphosphat Natriumtripolyphosphat ist.

7. Mundpflegezusammensetzung nach Anspruch 6, wobei das Natriumtripolyphosphat in einer Menge von 0,5 bis 5,0 Gew.-% bezogen auf das Gesamtgewicht der Zusammensetzung vorliegt.

8. Mundpflegezusammensetzung nach einem der vorhergehenden Ansprüche, wobei die Zusammensetzung ferner Glycerin umfasst, wobei das Glycerin in einer Gesamtmenge von 20 bis 50 % bezogen auf das Gesamtgewicht der Zusammensetzung vorliegt.

9. Mundpflegezusammensetzung nach einem der vorhergehenden Ansprüche, wobei das Verhältnis der Menge an Zinkoxid (Gew.-%) zu Zinkcitrat (Gew.-%) von 1,5:1 bis 4,5:1 beträgt.

10. Mundpflegezusammensetzung nach einem der vorhergehenden Ansprüche, die ein Aufhellungsmittel umfasst.

11. Mundpflegezusammensetzung nach einem der vorhergehenden Ansprüche, umfassend:
a. ungefähr 1,0 % Zinkoxid
b. ungefähr 0,5 % Zinkcitrat
c. ungefähr 4500 ppm Zinn(II)-fluorid;
d. ungefähr 3,0 % Natriumtripolyphosphat

12. Beliebige der vorstehenden Zusammensetzungen, umfassend:
a. ungefähr 1,0 % Zinkoxid
b. ungefähr 0,5 % Zinkcitrat
c. ungefähr 4500 ppm Zinn(II)-fluorid;
d. ungefähr 3,0 % Natriumtripolyphosphat; und
a. ungefähr 41% Glycerin

13. Mundpflegezusammensetzung nach einem der vorhergehenden Ansprüche, wobei die Mundpflegezusammensetzung eine der folgenden Mundpflegezusammensetzungen sein kann, die aus der Gruppe bestehend aus einer Zahnpasta oder einem Zahnputzmittel, einem Mundwasser oder einer Mundspülung, einem topischen Mundgel, einem Kaugummi und einem Gebissreiniger ausgewählt sind.

14. Mundpflegezusammensetzung nach einem der vorhergehenden Ansprüche, wobei die Zusammensetzung durch Kombinieren der Inhaltsstoffe gemäß einer der vorausgegangen Zusammensetzungen erzielt wird oder erzielt werden kann.

15. Mundpflegezusammensetzung nach einem der vorhergehenden Ansprüche zur Verwendung in einem Verfahren zum Verbessern der Mundgesundheit, das das Anwenden einer wirksamen Menge der Mundpflegezusammensetzung auf die Mundhöhle eines Subjekts umfasst, das diese benötigt.

## Revendications

1. Composition pour soins buccaux, comprenant :
a. une première source de zinc comprenant de l'oxyde de zinc ;
b. une seconde source de zinc comprenant du citrate de zinc ;
c. une source d'ions stanneux ; et
d. un polyphosphate ; dans laquelle
le citrate de zinc est en une quantité de 0,25 à 1 % en poids et l'oxyde de zinc est présent en une quantité de 0,75 à 1,25 % en poids sur la base du poids total de la composition.

2. Composition pour soins buccaux selon l'une quelconque des revendications précédentes, dans laquelle la source d'ions stanneux est du fluorure stanneux, du chlorure stanneux dihydraté, du pyrophosphate stanneux, des sels organiques de carboxylate stanneux tels que le formiate, l'acétate, le gluconate, le lactate, le tartrate, l'oxalate, le malonate et le citrate stanneux, ou un mélange de ceux-ci.

3. Composition pour soins buccaux selon la revendication 1, dans laquelle la source stanneuse est du fluorure stanneux, de préférence
dans laquelle le fluorure stanneux est présent en une quantité de 0,1 % en poids à 2 % en poids sur la base du poids total de la composition, ou
dans laquelle le fluorure stanneux est en une quantité de 50 à 25 000 ppm.

4. Composition pour soin buccaux selon l'une quelconque des revendications précédentes, dans laquelle la composition comprend un copolymère.

5. Composition pour soins buccaux selon la revendication 4, dans laquelle le copolymère est un copolymère de PVM/MA.

6. Composition pour soins buccaux selon l'une quelconque des revendications précédentes, dans laquelle le polyphosphate est le tripolyphosphate de sodium.

7. Composition pour soins buccaux selon la revendication 6, dans laquelle le tripolyphosphate de sodium est présent en une quantité de 0,5 à 5,0 % en poids, sur la base du poids total de la composition.

8. Composition pour soins buccaux selon l'une quelconque des revendications précédentes, dans laquelle la composition comprend en outre de la glycérine, dans laquelle la glycérine est en une quantité totale de 20 à 50 %, sur la base du poids total de la composition.

9. Composition pour soin buccaux selon l'une quelconque des revendications précédentes, dans laquelle le rapport entre la quantité d'oxyde de zinc (% en poids) et le citrate de zinc (% en poids) est de 1,5:1 à 4,5:1.

10. Composition pour soin buccaux selon l'une quelconque des revendications précédentes, comprenant un agent blanchissant.

11. Composition pour soin buccaux selon l'une quelconque des revendications précédentes, comprenant :
a. environ 1,0 % d'oxyde de zinc
b. environ 0,5 % de citrate de zinc
c. environ 4500 ppm de fluorure stanneux ;
d. environ 3,0 % de tripolyphosphate de sodium

12. Une quelconque des compositions précédentes, comprenant :
e. environ 1,0 % d'oxyde de zinc
f. environ 0,5 % de citrate de zinc
g. environ 4500 ppm de fluorure stanneux ;
h. environ 3,0 % de tripolyphosphate de sodium ; et
i. environ 41 % de glycérine

13. Composition pour soins buccaux selon l'une quelconque des revendications précédentes, dans laquelle la composition orale peut être l'une quelconque des compositions orales suivantes choisies dans le groupe constitué de : un dentifrice, un rince-bouche ou une eau dentaire, un gel oral topique, un chewing gum et un nettoyant pour prothèses dentaires.

14. Composition pour soins buccaux selon l'une quelconque des revendications précédentes, dans laquelle la composition est ou peut être obtenue en combinant les ingrédients comme indiqué dans l'une quelconque des compositions précédentes.

15. Composition pour soins buccaux selon l'une quelconque des revendications précédentes destinée à être utilisée dans un procédé pour améliorer la santé bucco-dentaire, comprenant l'application d'une quantité efficace de ladite composition pour soins buccaux dans la cavité buccale d'un sujet en ayant besoin.
